# EUROPÄISCHE PATENTSCHRIFT

(11) **EP 1 725 196 B1**
(45) Veröffentlichungstag und Bekanntmachung des Hinweises auf die Patenterteilung: **01.05.2013**
(21) Anmeldenummer: 05706557.5
(22) Anmeldetag: 08.03.2005
(51) Int. Cl.: A61F 5/44

(54) **DRAINAGEBEUTEL**
DRAINAGE SAC
POCHE DE DRAINAGE

(30) Priorität: 17.03.2004 CH 4662004
(43) Veröffentlichungstag der Anmeldung: 29.11.2006
(73) Patentinhaber: Medela Holding AG, 6340 Baar (CH)
(72) Erfinder: KOCH, Urs, CH-6404 Greppen (CH); KÜNZLER, Hansruedi, CH-8932 Mettmenstetten (CH); RÖLLIN, Richard, CH-6313 Menzingen (CH)
(74) Vertreter: Clerc, Natalia
(86) Internationale Anmeldenummer: PCT/CH2005/000139
(87) Internationale Veröffentlichungsnummer: WO 2005/087152

(56) Entgegenhaltungen:
- EP-A- 0 842 674
- WO-A-99/23978
- WO-A-2004/026166
- DE-U1- 29 603 879
- US-B1- 6 352 526

## Beschreibung

### Technisches Gebiet

Die Erfindung betrifft einen Drainagebeutel gemäss Oberbegriff des Patentanspruchs 1.

### Stand der Technik

Zum Absaugen von Körperflüssigkeiten im medizinischen Bereich, beispielsweise bei bzw. nach chirurgischen Eingriffen aber auch in der Wunddrainage, der Thoraxdrainage oder beim Absaugen von Körperfetten, werden Auffangbehälter verwendet, welche einerseits mit einer Saugpumpe verbunden sind und an welche andererseits eine mit einem Patienten verbundene Drainageleitung anschliesst. Mittels der Saugpumpe wird im Auffangbehälter ein Unterdruck erzeugt, so dass die Körperflüssigkeit durch die Drainageleitung abgesaugt und im Auffangbehälter gesammelt werden kann. Der Auffangbehälter ist üblicherweise aus einem starren Material gefertigt und verfügt über einen dicht verschliessbaren Deckel.

Um den heutigen Hygienevorschriften zu genügen, wird die Flüssigkeit jedoch üblicherweise nicht im starren Behälter selber, sondern in einem Einwegbeutel gesammelt, welcher im Auffangbehälter angeordnet ist. Ein derartig mit Flüssigkeit gefüllter Einwegbeutel, auch Drainagebeutel genannt, lässt sich einfach entfernen und einer geeigneten Entsorgung zuführen. Der Auffangbehälter wird gereinigt und wiedervenvendet.

Aus EP-A-0'861'688 ist ein derartiger Einwegbeutel zur Aufnahme von abzusaugender Flüssigkeit bekannt. Er weist einen starren Kopfteil auf, an welchem ein Anschlusselement für die Drainage- oder Absaugleitung und eine weitere Öffnung zur Verbindung mit der Saugquelle angeordnet ist. Dieser starre Kopfteil lässt sich an einem Deckel des Auffangbehälters befestigen, wobei das Anschlusselement für die Drainageleitung gleichzeitig als Befestigungsmittel zur Befestigung des Drainagebeutels am Deckel dient. Dieser Drainagebeutel hat sich in der Praxis bewährt.

Bei falscher Handhabung kann es jedoch bei gewissen Drainagebeuteln geschehen, dass bereits abgesaugte Flüssigkeit zum Patienten zurückgedrückt wird. Dies geschieht insbesondere dann, wenn am Ende eines Abpumpvorgangs nicht die Vakuumpumpe abgeschaltet wird, sondern die Leitung anderweitig unterbrochen wird, beispielsweise indem der Verbindungsschlauch zwischen Drainagebeutel und Vakuumpumpe herausgezogen wird.

Handelt es sich dabei um die eigene Körperflüssigkeit, kann dies bereits negative Folgen haben. Ein noch grösseres Hygienerisiko birgt jedoch die tägliche Praxis einzelner Spitäler: Aus Kostengründen wird nämlich oft ein Beutel, welcher von einem ersten Patienten noch nicht vollständig gefüllt worden ist, bei einem zweiten bzw. dritten Patienten benützt. Dabei wird lediglich die Drainageleitung ausgewechselt. Kommt es nun bei einem dieser nachfolgenden Patienten zu einer falschen Handhabung und somit zu einem Zurückfliessen der bereits im Beutel befindlichen Flüssigkeit, so kann dies schwere gesundheitliche Folgen nach sich ziehen.

DE 296 03 879 U offenbart einen Drainagebeutel gemäss Oberbegriff des Patentanspruchs 1.

### Darstellung der Erfindung

Es ist deshalb eine Aufgabe der Erfindung, eine Vorrichtung zu schaffen, welche dieses Problem behebt.

Diese Aufgabe löst ein Drainagebeutel mit den Merkmalen des Anspruchs 1.

Der erfindungsgemässe Drainagebeutel zur Aufnahme von abgesaugter Körperflüssigkeit, welcher ein Anschlusselement zur Verbindung mit einer Drainageleitung aufweist, ist mit einem Einwegventil versehen, welches einen Fluss der Körperflüssigkeit von der Drainageleitung in den Drainagebeutel erlaubt und welches einen Fluss der Körperflüssigkeit vom Drainagebeutel in die Drainageleitung verhindert.

Das Einwegventil ist auf einem Einlassstutzen des Drainagebeutels angeordnet, wobei es eine Zunge aufweist, welche eine Einlassöffnung dieses Stutzens überdeckt. Diese Ausbildung des Einwegventils ist kostengünstig, so dass sie die Produktionskosten der als Einwegbeutel benützten Drainagebeutel nicht wesentlich erhöht. Vorzugsweise bildet der Einlassstutzen die beutelinnenseitige Verlängerung des Anschlusselements.

In einer bevorzugten Ausführungsform weist der Einwegbeutel einen starren Kopfteil auf, an welchem das Anschlusselement angeordnet ist und an welchem sich vorzugsweise auch eine Öffnung zur Verbindung mit einer Saugquelle befindet, welche den für die Absaugung notwendigen Unterdruck erzeugt.

Weitere vorteilhafte Ausführungsformen gehen aus den abhängigen Patentansprüchen hervor.

### Kurze Beschreibung der Zeichnungen

Im folgenden wird der Erfindungsgegenstand anhand eines bevorzugten Ausführungsbeispiels, welches in den beiliegenden Zeichnungen schematisch dargestellt ist, erläutert. Es zeigen:
- Figur 1: eine perspektivische Darstellung eines erfindungsgemässen Drainagebeutels;
- Figur 2: eine perspektivische Darstellung eines starren Kopfteils des Drainagebeutels gemäss Figur 1 und
- Figur 3: eine perspektivische Darstellung eines Einwegventils gemäss Figur 1.

### Wege zur Ausführung der Erfindung

Zur besseren Erkennbarkeit der erfindungswesentlichen Merkmale ist der Drainagebeutel in den Figuren auf dem Kopf liegend dargestellt. Seine Gebrauchslage ist üblicherweise um 180° gedreht, dass heisst der nachfolgend beschriebene Anschlussnippel ragt nach oben und die Folie 1 nach unten.

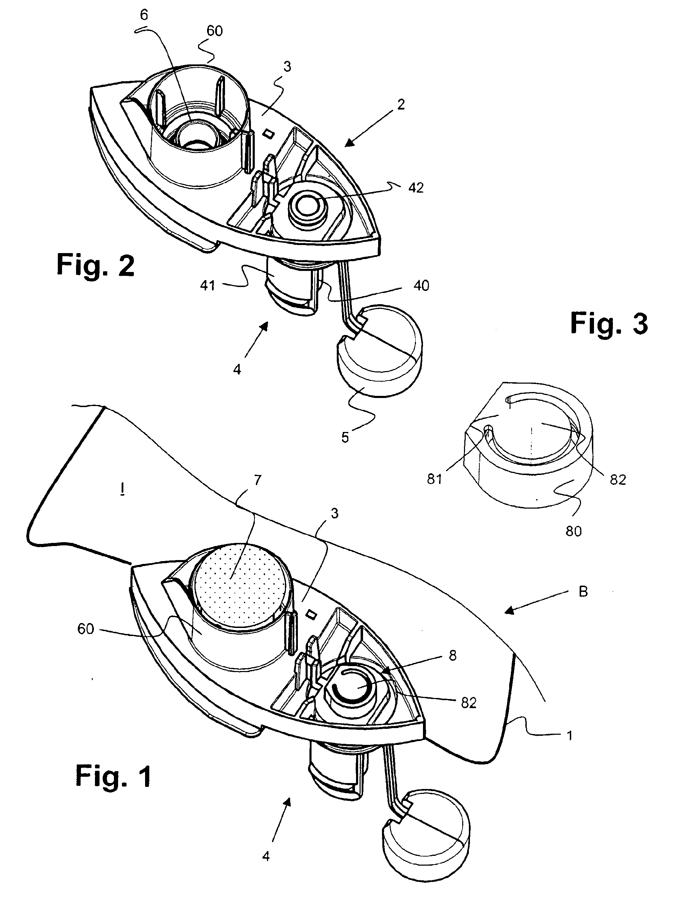

Figur 1 zeigt einen erfindungsgemässen Drainagebeutel B. Er besteht im wesentlichen aus einem biegsamen Material, insbesondere einer zu einem Beutel verschweissten Folie 1, und einem starren Kopfteil 2, welches mit der Folie 1 verschweisst ist. Der Drainagebeutel B ist bis auf im Kopfteil 2 vorhandene Öffnungen dicht verschlossen.

Die Folie 1 kann als Stehbeutel ausgebildet scin. Sie kann jedoch auch einfach aus zwei einander gegenüberliegenden Folienblättern bestehen, welche miteinander verschweisst sind. Ebenso kann sie als Schlauchbeutel ausgebildet sein, dessen eine Querseite mit einer Schweissnaht verschlossen ist und dessen andere gegenüberliegende Querseite mindestens teilweise das starre Kopfteil 2 umgibt.

Das starre Kopfteil 2 ist vorzugsweise aus einem Kunststoff gefertigt. Sie weist eine Basisplatte 3 auf, an welcher die Folie 1 angeschweisst ist. Wie in Figur 2 gut erkennbar ist, ist die Basisplatte 3 mit einer Durchgangsöffnung versehen, welche von einem Anschlusselement 4 umgeben ist. Das Anschlusselement 4 weist einen Anschlussnippel 40 auf, an welchem eine zum Patienten führende, hier nicht dargestellte Drainageleitung anschliessbar ist. Das Anschlusselement 4 verfügt ferner über Rastklinken 41, welche den Anschlussnippel 40 umgeben und welche zur lösbaren Befestigung des Drainagebeutels B in einem hier nicht dargestellten starren Drainagebehälter dienen. An der Basisplatte 3 ist ferner ein Verschlussdeckel 5 beweglich angelenkt, welcher vor und nach Gebrauch des Beutels B den Anschlussnippel 40 verschliesst. Vorzugsweise wird der Deckel 5 über die Rastklinken 41 gestülpt, wobei er einen inneren Stutzen aufweist, welcher dichtend in den Anschlussnippel 40 hineinragt.

Das starre Kopfteil 2 verfügt vorzugsweise ferner über eine Öffnung 6, welche zur Verbindung mit einer hier nicht dargestellten Saugquelle, insbesondere einer Saugpumpe, dient. Damit die Saugquelle vor der abgesaugten Körperflüssigkeit geschützt wird, ist diese Öffnung 6 mit einer Membran 7, beispielsweise einem hydrophoben Filter, verschlossen. Dieses Filter 7 ist vorzugsweise in einem in den Beutelinnenraum hineinragenden Saugstutzen 60 angeordnet.

Ähnliche Beutel sind in der eingangs erwähnten EP-A-0'861'668 beschrieben und dargestdellt.

Der erfindungsgemässe Drainagebeutel B weist jedoch zusätzlich ein Einwegventil 8 auf, welches einen Fluss der Körperflüssigkeit von der Drainageleitung in den Drainagebeutel erlaubt und welches einen Fluss der Körperflüssigkeit vom Drainagebeutel in die Drainageleitung verhindert.

In der hier dargestellten Ausführungsform verfügt das starre Kopfteil 2 hierfür über einen Einlassstutzen 42, auf welchem das Einwegventil 8 angeordnet ist. Dieser Einlassstutzen 42 ist hier die in den Beutelinnenraum 1 hineinragende Verlängerung des Anschlusselements 4, so dass das Einwegventil 8 auf einer der Drainageleitung abgewandten Seite des Anschlusselements 4 angeordnet ist.

Das Einwegventil, wie es in Figur 3 dargestellt ist, weist einen im wesentlichen zylinderrohrförmigen Grundkörper 80 auf, welcher den Einlassstutzen 42 umschliesst. An diesem Grundkörper 80 ist über einen Verbindungssteg 81 eine flache Zunge 82 beweglich angeordnet, welche die Einlassöffnung des Einlassstutzens 42 überdeckt und auf derer oberen Stirnfläche aufliegt. Durch den im Beutelinnenraum 1 erzeugten Unterdruck wird die Zunge 82 von der Öffnung des Einlassstutzens 42 weggezogen und gibt die Öffnung für die hineinfliessende abgesaugte Körperflüssigkeit frei. Der plane Steg 81 erleichtert dabei die Öffnungsbewegung. Liegt im Drainagebeutelinnenraum I aber kein Unterdruck gegenüber der Atmosphäre bzw. der Umgebung vor, liegt die Zunge 82 dichtend auf dem Einlassstutzen 42 auf. Vorzugsweise ist dieses Einwegventil 8 aus einem leicht elastischen Material, insbesondere einem relativ steifen Gummi, hergestellt. Dadurch lässt es sich auf einfache Art und Weise über den Einlassstutzen 42 stülpen.

Verfügt das Kopfteil 2 wie hier dargestellt über den Saugstutzen 60, so ist der Einlassstutzen 42 vorzugsweise kürzer ausgebildet als der Saugstutzen 60, so dass letzterer weiter in den Beutelinnenraum I hineinragt. Des weiteren ist die Zunge 82 des Ventils 8 vorzugsweise so angeordnet, dass der Verbindungssteg 81 dem Saugstutzen 60 zugewandt ist. Diese Massnahmen ermöglichen jeweils einen Spritzschutz, so dass die Membran 7 möglichst nicht mit Flüssigkeit in Kontakt kommt und so weniger rasch verstopfen kann.

Vorzugsweise ist zudem eine Verdrehsicherung vorhanden, damit das Einwegventil 8 lagedefiniert angeordnet werden kann. Im hier dargestellten Beispiel weist deshalb der Grundkörper 80 eine im wesentlichen kreisförmige Innenwandung auf, welche im Bereich des Verbindungsstegs 81 abgeflacht ist und somit bei Anlage an den kreisförmigen Mantel des Einlassstutzens 42 eine Verdrehsicherung bildet. Alternativ lässt sich eine entsprechende Abflachung auch auf den äusseren Mantel des Ventils 8 anordnen.

Des weiteren können Rückhaltemittel vorhanden sein, um das Einwegventil 8 in seiner Position zu halten. Beispielsweise kann der Einlassstutzen 42 mit Rückhaltenoppen, einer Wulst oder mit Rückhalterippen versehen sein. Er kann auch konisch ausgebildet sein.

Der oben beschriebene Drainagebeutel B ist ein bevorzugtes Ausführungsbeispiel. Es lassen sich jedoch auch andere Drainagebeutel mit einem derartigen Einwegventil versehen. Beispielsweise kann das Einwegventil auch auf der Aussenseite des Beutels angeordnet sein. Der Beutel muss nicht zwingend über ein starres Kopfteil verfügen. Der Beutel muss auch nicht zwingend über die Öffnung zur Verbindung mit einer Saugquelle verfügen. Ferner kann der Beutel noch weitere Öffnungen, beispielsweise Öffnungen zur erleichterten Entleerung des Beutels, aufweisen.

Der erfindungsgemässe Drainagebeutel verhindert auf einfache Weise ein Rückfliessen der bereits abgesaugten Flüssigkeit und erfüllt höchste Anforderungen an die Hygiene.

### Bezugszeichenliste

- B: Drainagebeutel
- I: Beutelinnenraum

- 1: Folie

- 2: Kopfteil

- 3: Basisplatte

- 4: Anschlusselement
- 40: Anschlussnippel
- 41: Rastklinken
- 42: Einlassstutzen

- 5: Versehlussdeckel

- 6: Öffnung
- 60: Saugstutzen

- 7: Membran

- 8: Einwegventil
- 80: Grundkörper
- 81: Verbindungssteg
- 82: Zunge

## Patentansprüche

1. Drainagebeutel zur Aufnahme von abgesaugter Körperflüssigkeit, wobei der Drainagebeutel ein Anschlusselement (4) zur Verbindung mit einer Drainageleitung aufweist, wobei der Drainagebeutel mit einem Einwegventil (8) versehen ist, welches einen Fluss der Körperflüssigkeit von der Drainageleitung in den Drainagebeutel erlaubt und welches einen Fluss der Körperflüssigkeit vom Drainagebeutel in die Drainageleitung verhindert, **dadurch gekennzeichnet, dass** der Drainagebeutel einen Einlassstutzen (42) aufweist, wobei das Einwegventil (8) auf diesem Einlassstutzen (42) angeordnet ist, dass das Einwegventil (8) einen zylinderrohrförmigen Grundkörper (80) aufweist, welcher den Einlassstutzen (42) umschliesst, und dass das Einwegventil (8) ferner eine am zylinderförmigen Grundkörper (80) beweglich angeordnete Zunge (82) aufweist, welche eine Einlassöffnung des Einlassstutzens (42) überdeckt..

2. Drainagebeutel nach Anspruch 1, wobei der Drainagebeutel ein starres Kopfteil (2) aufweist und wobei das Anschlusselement (4) im starren Kopfteil (2) angeordnet ist.

3. Drainagebeutel nach einem der Ansprüche 1 oder 2, wobei das Einwegventil (8) am Anschlusselement (4) angeordnet ist.

4. Drainagebeutel nach einem der Ansprüche 1 bis 3, wobei das Einwegventil (8) auf einer der Drainageleitung abgewandten Seite des Anschlusselements (4) angeordnet ist.

5. Drainagebeutel nach Anspruch 4, wobei der Einlassstutzen (42) mit Rückhal temittel versehen ist.

6. Drainagebeutel nach einem der Ansprüche 1 bis 5, wobei der Drainagebeutel eine Öffnung (6) zur Verbindung mit einer Saugquelle aufweist.

7. Drainagebeutel nach den Ansprüchen 2 und 6, wobei diese Öffnung (6) im starren Kopfteil (2) angeordnet ist.

8. Drainagebeutel nach Anspruch 2, wobei der Drainagebeutel mit Ausnahme des starren Kopfteils (2) aus einem biegsamen Material, insbesondere einer zu einem Beutel geformten Kunststofffolie, gefertigt ist.

9. Drainagebeutel nach Anspruch 6, wobei die Öffnung (6) zur Verbindung mit der Saugquelle einen in einen Innenraum (I) des Drainagebeutels hineinragenden Saugstutzen (60) aufweist, wobei der Einlassstutzen (42) kürzer ausgebildet ist als der Saugstutzen (60).

## Claims

1. A drainage bag for receiving body fluid removed by suction, wherein the drainage bag comprises a connecting element (4) for connection to a drainage line, wherein the drainage bag comprises a one-way valve (8) that allows the body fluid to flow from the drainage line to the drainage bag and that prevents any flow of the body fluid from the drainage bag to the drainage line, **characterised in that** the drainage bag comprises an inlet nozzle (42), wherein the one-way valve (8) is arranged on this inlet nozzle (42); **in that** the one-way valve (8) comprises a cylindrical base body (80) that encompasses the inlet nozzle (42); and **in that** the one-way valve (8) furthermore comprises a tongue (82) that is movably arranged on the cylindrical base body (80), which tongue (82) covers an inlet opening of the inlet nozzle (42).

2. The drainage bag according to claim 1, wherein the drainage bag comprises a rigid head section (2), and wherein the connecting element (4) is arranged in the rigid head section (2).

3. The drainage bag according to one of claims 1 or 2, wherein the one-way valve (8) is arranged on the connecting element (4).

4. The drainage bag according to any one of claims 1 to 3, wherein the one-way valve (8) is arranged on a side of the connecting element (4), which side faces away from the drainage line.

5. The drainage bag according to claim 4, wherein the inlet nozzle (42) comprises retention means.

6. The drainage bag according to any one of claims 1 to 5, wherein the drainage bag comprises an opening (6) for connection to a suction source.

7. The drainage bag according to claims 2 and 6, wherein this opening (6) is arranged in the rigid head section (2).

8. The drainage bag according to claim 2, wherein the drainage bag, except for its rigid head section (2), is made from a flexible material, in particular a plastic foil shaped to form a bag.

9. The drainage bag according to claim 6, wherein the opening (6) for connection to the suction source comprises a suction nozzle (60) that projects into the interior (I) of the drainage bag, wherein the inlet nozzle (42) is designed so as to be shorter than the suction nozzle (60).

## Revendications

1. Poche de drainage destinée à recevoir du liquide corporel aspiré, où la poche de drainage comprend un élément de connexion (4) pour la connexion à une ligne de drainage, où la poche de drainage comprend un clapet unidirectionnel (8), qui permet un écoulement du liquide corporel de la ligne de drainage dans la poche de drainage et qui empêche un écoulement du liquide corporel de la poche de drainage dans la ligne de drainage, **caractérisée en ce que** la poche de drainage comprend une tubulure d'admission (42), où le clapet unidirectionnel (8) est disposé sur cette tubulure d'admission (42), **en ce que** le clapet unidirectionnel (8) comprend un corps de base (80) formant un tube cylindrique, qui entoure la tubulure d'admission (42) et **en ce que** le clapet unidirectionnel (8) comprend en outre une languette (82) disposée de manière mobile sur le corps de base (80) formant un tube cylindrique, laquelle recouvre une ouverture d'admission de la tubulure d'admission (42).

2. Poche de drainage selon la revendication 1, où la poche de drainage comprend une partie de tête rigide (2) et où l'élément de connexion (4) est disposé dans la partie de tête rigide (2).

3. Poche de drainage selon la revendication 1 ou 2, où le clapet unidirectionnel (8) est disposé sur l'élément de connexion (4).

4. Poche de drainage selon l'une quelconque des revendications 1 à 3, où le clapet unidirectionnel (8) est disposé sur un côté de l'élément de connexion (4) qui est opposé de la ligne de drainage.

5. Poche de drainage selon la revendication 4, où la tubulure d'admission (42) comprend des moyens de retenue.

6. Poche de drainage selon l'une quelconque des revendications 1 à 5, où la poche de drainage comprend une ouverture (6) pour la connexion à une source d'aspiration.

7. Poche de drainage selon les revendications 2 et 6, où cette ouverture (6) est disposée dans la partie de tête rigide (2).

8. Poche de drainage selon la revendication 2, où la poche de drainage étant fabriquée, à l'exception de la partie de tête rigide (2), dans un matériau flexible, en particulier dans un film plastique formé en poche.

9. Poche de drainage selon la revendication 6, où l'ouverture (6) pour la connexion à une source d'aspiration comprend une tubulure d'aspiration (60) faisant saillie dans un espace intérieur (I) de la poche de drainage, où la tubulure d'admission (42) est plus courte que la tubulure de succion (60).
